# FASCICULE DE BREVET EUROPEEN

(11) **EP 4 175 581 B1**
(45) Date de publication et mention de la délivrance du brevet: **20.11.2024**
(21) Numéro de dépôt: 21735986.8
(22) Date de dépôt: 02.07.2021
(51) Int. Cl.: A61B 34/32, A61B 90/00, A61B 34/10

(54) **DISPOSITIF ROBOTIQUE POUR LE GUIDAGE D'UN BRAS ROBOTISE**
ROBOTISCHE VORRICHTUNG ZUR FÜHRUNG EINES ROBOTERARMS
ROBOTIC DEVICE FOR GUIDING A ROBOTIC ARM

(30) Priorité: 03.07.2020 FR 2007102
(43) Date de publication de la demande: 10.05.2023
(73) Titulaire: Squaremind, 75010 Paris (FR)
(72) Inventeur: SERRAT, Tanguy, 92210 Saint-Cloud (FR); KHACHLOUF, Ali, 75010 Paris (FR)
(74) Mandataire: Oak & Fox
(86) Numéro de dépôt international: PCT/EP2021/068319
(87) Numéro de publication internationale: WO 2022/003154

(56) Documents cités:
- WO-A2-2019/058315
- FR-A1- 2 917 598
- FR-A1- 2 917 598
- FR-A1- 3 073 135
- FR-A1- 3 073 135
- US-A1- 2008 033 410
- US-A1- 2008 033 410
- US-A1- 2016 191 887
- US-A1- 2016 191 887
- ZHANG ET AL: "Recent progresses on real-time 3D shape measurement using digital fringe projection techniques", OPTICS AND LASERS IN ENGINEERING, ELSEVIER, AMSTERDAM, NL, vol. 48, no. 2, 1 February 2010 (2010-02-01), pages 149 - 158, XP026756323, ISSN: 0143-8166, [retrieved on 20090416], DOI: 10.1016/J.OPTLASENG.2009.03.008

## Description

### DOMAINE

Le domaine de l'invention concerne celui des dispositifs robotisés permettant de guider un bras articulé dans l'espace pour le traitement d'une zone d'un corps d'un patient. Plus précisément, le domaine de l'invention se rapporte à un dispositif permettant d'asservir les mouvements d'un bras robotisé selon une analyse d'images acquises en temps réel.

### ETAT DE L'ART

Actuellement, il existe un besoin de définir un dispositif permettant de diagnostiquer ou traiter par une analyse d'images ou une émission d'un signal une zone d'un corps d'un patient, ledit traitement ou ledit diagnostic étant réalisé en temps réel.

Un problème des solutions actuelles est qu'elles impliquent soit une installation importante d'un matériel optique permettant d'obtenir des images de différents points de vue afin de reconstituer une image 3D complète d'un sujet, soit de limiter l'ensemble des prises de vues en limitant l'installation d'un système optique avec la conséquence de limiter l'étendue de l'analyse pour conserver des performances d'analyse temps réel.

Dans le premier cas, le système impose une calibration de toutes les optiques et nécessite une mise en oeuvre de moyens importants de calculs des images acquises pour générer des trajectoires en temps réel.

Dans le second cas, le système ne peut restituer une vue complète et impose donc un protocole complexe et plus long pour diagnostiquer ou interagir sur la surface d'un corps. Un problème se pose alors de rétablir une configuration de prises d'image qui puissent être compatibles d'une précédente configuration lorsque l'analyse impose une segmentation du traitement en plusieurs temps de différentes parties du sujet.

US2008033410 A1 divulgue un dispositif robotique avec un système d'acquisition d'images selon l'état de la technique.

Il existe un besoin d'une solution compacte permettant la reconstruction d'un corps humain d'un patient pour interagir en temps réel avec un dispositif robotisé.

### RESUME DE L'INVENTION

L'invention ci-après détaillée permet de pallier les inconvénients précités. L'invention est définie par la revendication 1, autres formes de réalisation sont décrites dans les revendications dépendantes.

Selon un aspect, l'invention concerne un dispositif robotique assurant le guidage automatique d'un bras robotisé, comprenant un bâti maintenant un bras robotisé articulé selon une pluralité d'axes de liberté, ledit bras robotisé comportant un dispositif opérateur distal pour générer une interaction homme-machine, ledit dispositif robotique comportant, en outre, un système d'acquisition d'images comportant aux moins deux dispositifs optiques solidaires dudit bâti et agencés en au moins deux positions distinctes du bâti, chaque dispositif optique étant configuré pour acquérir une image 3D, le système d'acquisition d'images étant configuré pour acquérir une pluralité d'images 3D d'un corps humain d'un patient provenant d'au moins des deux dispositifs optiques, le dispositif robotique comportant une unité de calcul pour générer en temps réel un modèle de corps humain dudit patient à partir desdites images 3D acquises et une trajectoire de guidage référencée à la surface dudit modèle de corps humain, les mouvements dudit bras robotisé étant asservis sur ladite trajectoire de guidage.

Un avantage est d'obtenir un champ de vue suffisant pour guider un bras robot à la surface d'un corps humain. L'agencement d'un double système de caméra 3D sur un même bâti permet d'obtenir une référence fiable pour la calibration du système optique et du bras robot.

Selon un mode de réalisation, le système d'acquisition d'images comporte au moins 4 dispositifs optiques solidaires dudit bâti et agencés en au moins 4 positions distinctes dudit bâti. Selon un mode de réalisation, le système d'acquisition d'images comporte au moins 6 dispositifs optiques solidaires dudit bâti et agencés en au moins 6 positions distinctes dudit bâti. Selon un exemple de réalisation, chaque dispositif optique est configuré pour acquérir une image 3D. Un avantage de la multiplication des dispositifs optiques est de générer un nuage de points le plus complet possible de la surface du corps d'un patient. Un deuxième avantage se trouve dans la multiplication des points de vue et donc la réduction des effets d'occlusions d'objets pouvant se trouver entre le robot et le système de capture, cela peut-être le robot lui-même, les bras d'un opérateur, etc. Selon un exemple de réalisation, les dispositifs optiques sont désactivables individuellement ou par paire. A titre d'exemple, un actionneur ou une consigne tactile permet de désactiver les dispositifs optiques inférieurs situés au niveau de la partie inférieure du bâti lorsque le patient est en position allongée.

Selon un mode de réalisation, au moins un premier dispositif optique comprend un premier agencement linéaire d'au moins deux caméras selon un premier axe. Selon un mode de réalisation, au moins un second dispositif optique comprend un second agencement linéaire d'au moins deux caméras selon un second axe, ledit premier axe et ledit second axe étant non colinéaires entre eux. Un avantage est de permettre de générer un champ de vue adapté à générer une modélisation d'un corps d'un patient debout en prenant en considération la taille de l'individu et son envergure. Selon un autre mode, ils ne sont pas colinéaires entre eux.

Selon un mode de réalisation, au moins un dispositif optique comprend un moyen d'orientation de l'axe d'acquisition par l'actionnement d'une liaison pivot.

Selon un mode de réalisation, chaque dispositif optique comprend :
▪ au moins deux caméras infrarouges et/ou ;
▪ au moins une caméra couleur et/ou ;
▪ au moins une paire de caméras couleur configurées pour générer les images 3D par stéréoscopie et/ou ;
▪ au moins trois caméras comprenant deux caméras infrarouges et une caméra couleur et/ou ;
▪ au moins un projecteur infrarouge projetant un motif et deux caméras infrarouges capturant ledit motif projeté à la surface d'un corps humain.
▪ au moins un projecteur de lumière projetant des motifs de lumière structurée et au moins une caméra capturant lesdits motifs et/ou;
▪ une caméra temps de vol et/ou ;
▪ un dispositif émetteur-récepteur laser.

Selon un mode de réalisation, le système optique comporte :
▪ un premier ensemble de dispositifs optiques comportant un premier champ de vue compris entre 30° et 70° verticalement et entre 50 et 100° horizontalement et ;
▪ un second ensemble de dispositifs optiques comportant un second champ de vue compris entre 50° et 100° verticalement et entre 30 et 70° horizontalement.

Un avantage est de réduire l'occlusion générée par les déplacements du bras robot devant le système optique susceptible de générer des ombres. Selon un mode de réalisation, chaque dispositif optique comporte un champ de vue rectangulaire.

Selon un mode de réalisation, les dispositifs optiques sont agencés pour couvrir un champ de vue rapporté au système optique ayant une origine virtuelle déplacée hors du plan comportant les origines des dispositifs optiques. Ainsi, en se situant dernière les origines optiques, c'est-à-dire à une distance plus éloignée du sujet, l'origine du champ de vue virtuel offre la possibilité d'optimiser la construction d'un champ optique. Il est possible d'obtenir un champ de vue virtuel ayant une couverture permettant une vue plus large ou plus séparée grâce à un agencement des dispositifs optiques distants entre eux. Un avantage est de permettre d'acquérir des images d'un patient en position debout ou allongée.

Selon un mode de réalisation, au moins deux dispositifs optiques sont distancés dans un plan horizontal d'un repère cartésien d'une distance comprise dans la gamme [30cm et 100cm]. Selon un mode de réalisation, au moins deux dispositifs optiques sont distancés selon un axe vertical d'un repère cartésien d'une distance comprise dans la gamme [30cm et 100cm]. Un intérêt est de couvrir une large surface, de multiplier les points de vue et de réduire le bruit et les occlusions causant des acquisitions incomplètes.

Selon un mode de réalisation, les dispositifs optiques sont agencés pour couvrir un champ de vue couvert par un angle solide d'une valeur supérieure ou égale à 3·pi/2.

Selon un mode de réalisation, le bâti comprend un écran permettant d'afficher une image 3D du corps d'un individu à partir d'au moins un dispositif optique. Un avantage est de positionner un patient de manière optimale en ayant un retour direct de sa position à l'écran. Un autre avantage est d'effectuer des opérations de calibrations ou des réglages des orientations des dispositifs optiques. Selon un mode de réalisation, cet affichage est réalisé en temps réel afin de représenter l'image du patient et donc son positionnement dans l'espace sur l'écran.

Selon un mode de réalisation, le bâti comprend un corps principal et une embase, ledit corps principal maintenant le système optique et ladite embase étant pourvue de moyens de déplacement pour rendre le dispositif robotique mobile.

Selon un mode de réalisation, l'embase comporte un contrepoids, une pluralité de roulettes et au moins un frein actionnable pour stabiliser ledit dispositif robotique à une position fixe. Un avantage est de permettre une flexibilité de positionnement du dispositif robotique dans de nombreux lieux et qui ne nécessite pas une pré-installation d'un système optique et d'une console d'exploitation.

Selon un mode de réalisation, le bâti comporte des moyens de pivotement du corps principal vis-à-vis de l'embase et un moyen pour bloquer le pivotement dudit corps principal de sorte à orienter ledit corps principal vis-à-vis de l'embase d'un angle souhaité.

Selon un mode de réalisation, le corps principal comporte des ailettes symétriques de part et d'autre de l'axe vertical du corps principal pour maintenir les dispositifs d'acquisition, au moins une ailette comportant une poignée. Un avantage est d'augmenter l'envergure du dispositif robotique au niveau du positionnement des optiques afin d'élargir le champ de vue du système optique.

Selon un mode de réalisation, le dispositif robotique comprend au moins un dispositif électronique pour générer à partir des images acquises par le système d'acquisition un nuage de points représentant une surface tridimensionnelle du corps d'un patient. Un avantage est de générer des données qui puissent être traitées facilement par un réseau de neurones ou tout autre algorithme afin que ce dernier puisse générer un nuage de points, un maillage tridimensionnel, ou un modèle 3D d'un corps, facilement exploitable pour guider un bras robot.

Selon un mode de réalisation, le dispositif électronique génère un nuage de point global à partir d'une pluralité de nuage de points générés à partir de chaque image 3D acquise par chaque dispositif optique, ledit nuage de points global comportant une compilation de points de chaque nuage de points. Un avantage est de combler les portions de nuages de points du corps humain non acquises par un dispositif optique du fait d'une occlusion de son champ de vue sur une portion de l'espace. Un autre avantage est d'étendre au maximum la portion du corps d'un patient qui sera modélisée.

Selon un mode de réalisation, le bras robotisé comprend au moins 5 portions, chaque portion s'étendant selon un axe et pivotant par rapport à au moins un axe d'une autre portion au moyen d'une liaison pivot, les pivotements de l'ensemble des liaisons pivots entrainant une cinématique du bras robotisé. Un avantage est de permettre un large champ d'applications notamment en dermatologie ou en application de traitement des tatouages et plus généralement des applications dans le domaine médical non invasif et invasif.

Selon un mode de réalisation, le dispositif électronique génère à partir des images acquises par le système d'acquisition des consignes de guidage de la cinématique du bras robotisé.

Selon un mode de réalisation, le dispositif électronique génère un premier modèle 3D d'une surface de tout ou partie d'un corps humain, ledit premier modèle 3D comportant un maillage, ledit premier modèle 3D étant utilisé pour calculer une première trajectoire de guidage à la surface du premier modèle 3D, et pour calculer une première cinématique du bras robotisé. Un avantage est de générer un modèle du corps d'un patient pour planifier une trajectoire en vue d'un déplacement et d'un guidage du bras robot.

Selon un mode de réalisation, le dispositif électronique génère en temps réel, à partir des images acquises par le système d'acquisition, un nouveau modèle 3D d'une surface de tout ou partie d'un corps humain, ledit modèle 3D comportant un maillage, ledit modèle 3D généré à des instants réguliers étant utilisé pour recalculer en temps réel une nouvelle trajectoire de guidage à la surface du modèle 3D, et pour recalculer en temps réel une nouvelle cinématique du bras robotisé. Un avantage est de réactualiser le calcul de la trajectoire, notamment dans un intervalle de temps inférieur à 30ms.

Selon un mode de réalisation, le dispositif électronique comprend une autocalibration des dispositifs optiques du système d'acquisition par rapport à un dispositif optique de référence dudit système d'acquisition. Le dispositif de référence peut avantageusement être choisi comme référence de calibration du positionnement du bras robot. Ainsi, les nuages de points sont rapportés dans un même repère commun grâce aux différentes transformations géométriques calculées par un processus de calibration.

### BRÈVE DESCRIPTION DES FIGURES

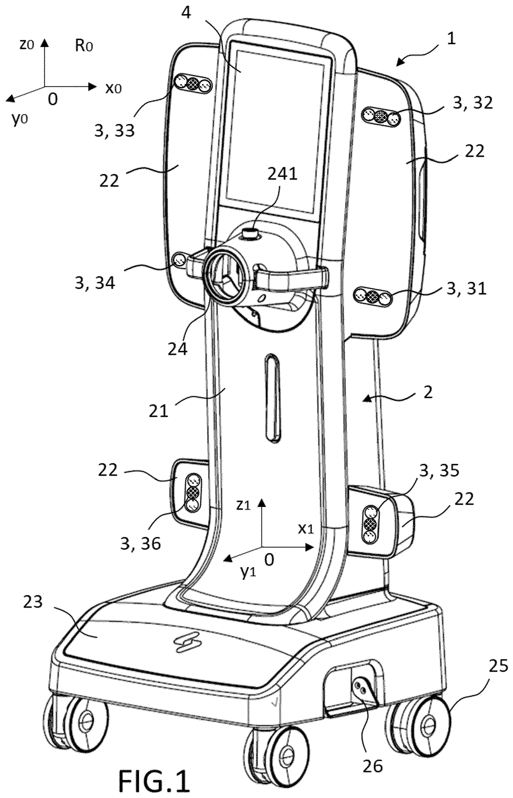

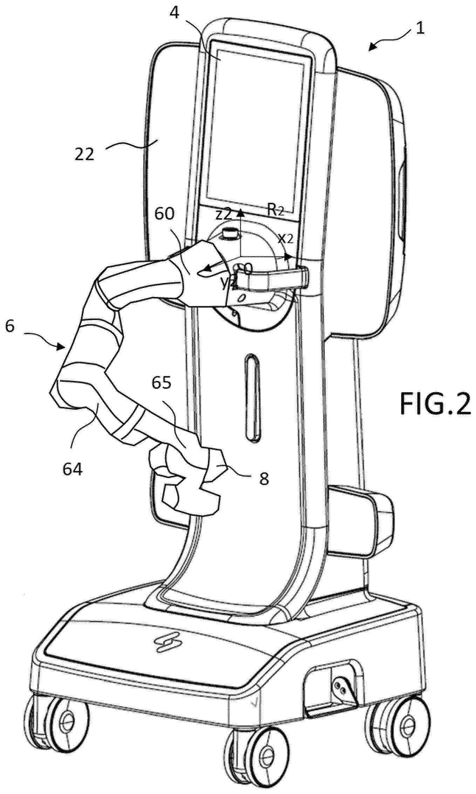

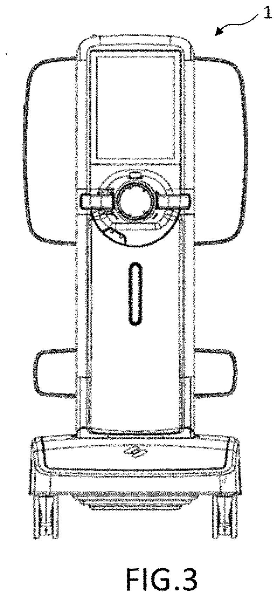

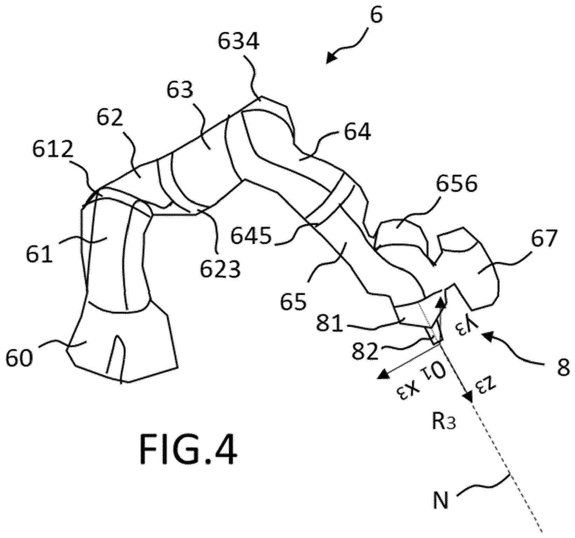

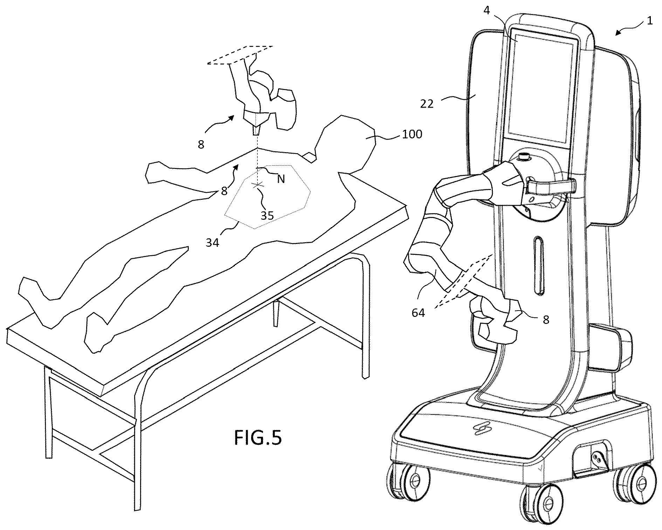

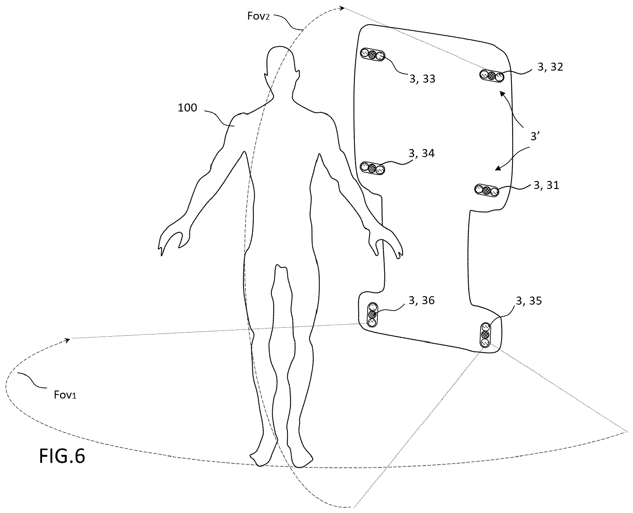

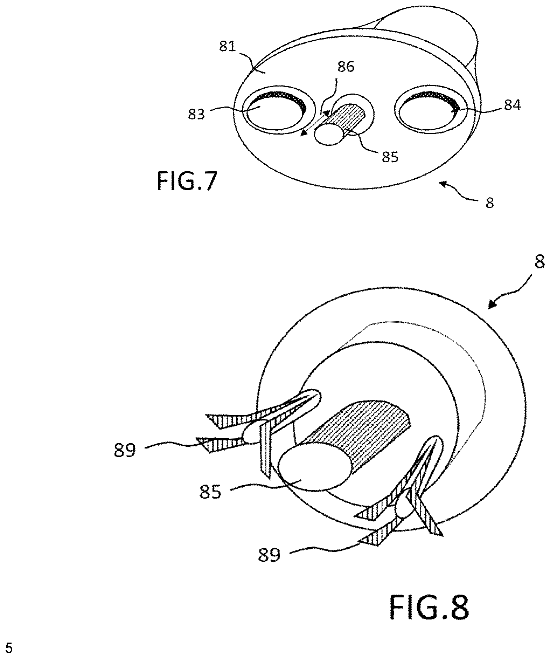

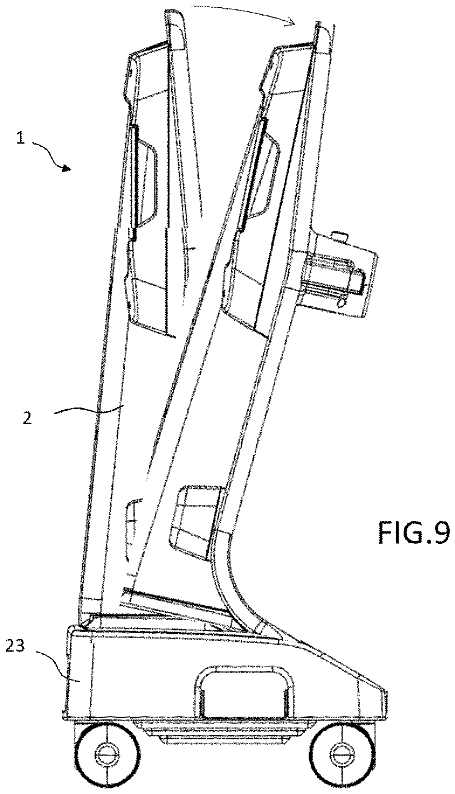

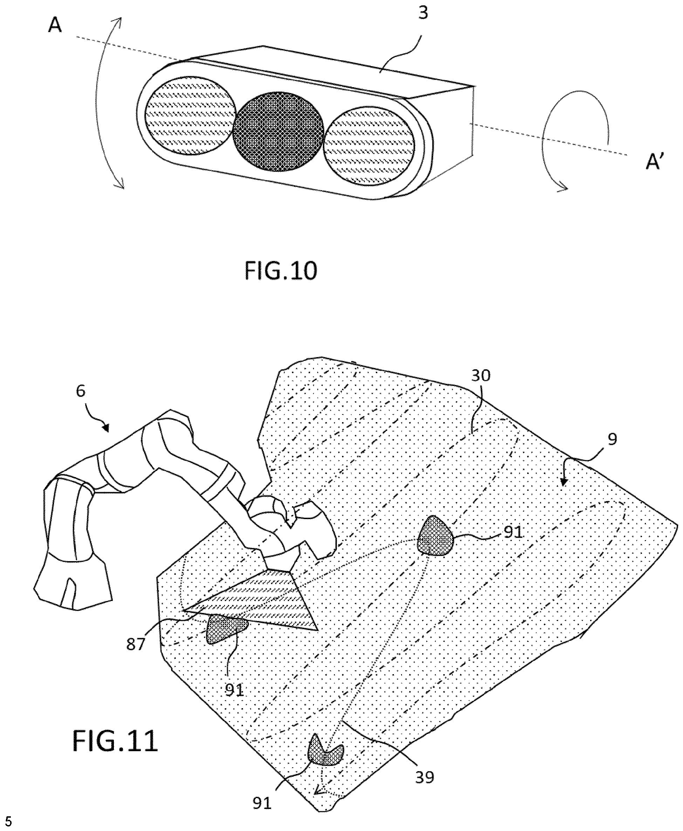

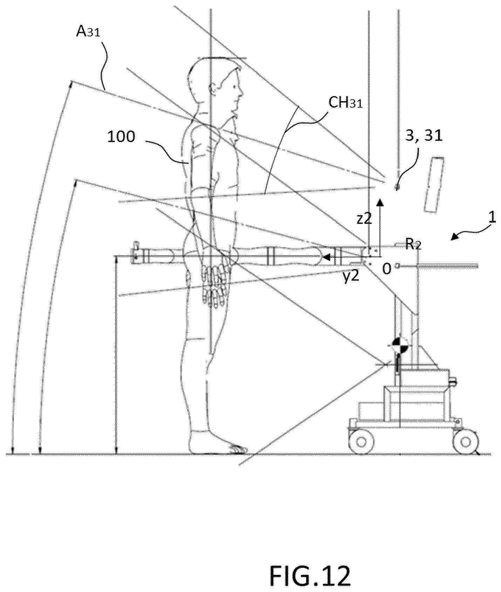

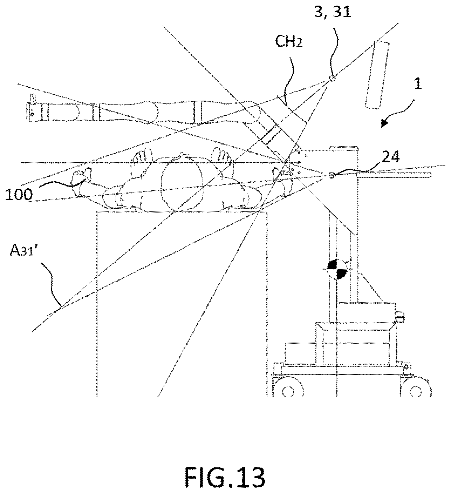

D'autres caractéristiques et avantages de l'invention ressortiront à la lecture de la description détaillée qui suit, en référence aux figures annexées, qui illustrent :
▪ [Fig. 1] : figure 1 : un dispositif robotique selon un mode de réalisation de l'invention sans bras robotisé articulé ;
▪ [Fig. 2] : figure 2 : un dispositif robotique selon un mode de réalisation de l'invention muni d'un bras robotisé articulé.
▪ [Fig. 3] : figure 3 : une vue de face d'un dispositif robotique selon un mode de réalisation de l'invention sans bras robotisé articulé ;
▪ [Fig. 4] : figure 4 : un bras robotisé adapté à être fixé sur un dispositif robotique selon un mode de l'invention ;
▪ [Fig. 5] : figure 5 : un exemple d'agencement d'un dispositif robotique d'un mode de réalisation de l'invention au côté d'une table d'intervention sur laquelle un patient est installé ;
▪ [Fig. 6] : figure 6 : un exemple d'agencement du système d'acquisition permettant d'obtenir un champ de vue pour acquérir la surface d'un corps d'un patient debout ;
▪ [Fig. 7] : figure 7 : un premier exemple de tête d'un bras robotisé du dispositif robotique de l'invention ;
▪ [Fig. 8] : figure 8 : un second exemple de tête d'un bras robotisé du dispositif robotique de l'invention ;
▪ [Fig. 9] : figure 9 : un exemple d'un dispositif de l'invention basculant pour adapter un champ de vue à une situation donnée ;
▪ [Fig. 10] : figure 10 : un exemple de dispositif optique orientable selon de l'invention ;
▪ [Fig. 11] : figure 11 : un exemple de trajectoire planifiée à la surface du corps d'un patient qui est recalculée en temps réel pour asservir les déplacements d'un bras robot de l'invention ;
▪ [Fig. 12] : figure 12 : un exemple d'agencement du dispositif robotique de l'invention permettant de parcourir la surface d'un corps d'un patient dans une position debout,
▪ [Fig. 13] : figure 13 : un exemple d'agencement du dispositif robotique de l'invention permettant de parcourir la surface d'un corps d'un patient dans une position allongée.

Le dispositif robotique 1 de l'invention vise à permettre une interaction d'un bras robotisé 6 articulé avec un corps humain 100. L'interaction est assurée par un dispositif opérateur distal 8 du bras robotisé 6 et correspond, par exemple, à l'émission d'un signal, à la prise d'une image ou encore à la génération d'un contact haptique sur la peau. D'autres interactions avec la surface ou à une profondeur proche de la surface d'un corps d'un patient sont possibles. Le dispositif robotique de l'invention permet de guider dans l'espace les mouvements du bras robotisé 6 articulé afin de permettre une interaction sur toute ou partie du corps humain selon une planification prédéfinie préalablement.

Un objectif de l'invention est de permettre d'acquérir des images d'un corps humain 100 selon un champ de vue adapté pour générer un modèle de corps humain afin de guider en temps réel le bras robotisé 6. Cet objectif est atteint par l'invention grâce notamment à un agencement optimisé d'un système d'acquisition d'images 3'. A cet effet, un dispositif robotique 1 compact et mobile est décrit. Un tel dispositif robotique 1 comprend par exemple des moyens de stabilisation du dispositif robotique 1 pour garantir un point de référence fiable et des mobilités de composants, tels que des optiques ou un bras articulé 6, dudit dispositif robotique 1 offrant une grande variété de configurations d'usages.

En outre, un avantage de l'invention est de fournir un dispositif robotique 1 amovible, rapidement déployable selon différentes configurations d'usage et ayant un encombrement réduit.

La figure 1 représente un mode de réalisation d'un dispositif robotique 1 de l'invention.

Selon un exemple de réalisation, le bâti 2 du dispositif robotique 1 de l'invention comprend une partie supérieure 21 comprenant un support de fixation 24 d'un bras robotisé 6, un écran 4, un système d'acquisition d'images 3' comportant aux moins deux dispositifs optiques 3. Dans le mode de réalisation de la figure 1, 6 dispositifs optiques 31, 32, 33, 34, 35, 36 sont représentés. En outre, le dispositif robotique 1 comprend une partie inférieure formant une embase 23 supportant la partie supérieure 21 et de la rendre amovible.

Selon un mode de réalisation, le dispositif robotique 1 de l'invention comprend une pluralité de bras robotisés 6. Dans ce cas, ils sont par exemple fixés à différents supports de fixation 24 d'un même bâti 2.

### Embase - Moyen de déplacement

Selon ce mode de réalisation, le dispositif robotique 1 comprend un bâti 2 amovible dans l'espace grâce à un moyen de déplacement représenté sur la figure 1 par une pluralité de roulettes 25 fixées à une embase 23 dudit bâti 2. Selon d'autres variantes, le moyen de déplacement peut être alternativement des rails guidés dans des rainures prédéfinies d'une surface d'un sol ou d'un mur ou inversement des rainures coopérant avec des rails disposés à la surface d'un sol ou d'un mur. Selon un exemple de cette variante, des rails disposés horizontalement le long d'un mur ou d'une cloison ont un profil incurvé permettant de maintenir le bras robot 1 et de le faire circuler sur une portion d'angle de 90°. Dans le cas où le dispositif robotique 1 est déplaçable le long d'une paroi verticale, le dispositif robotique 1 de l'invention peut ne pas comprendre d'embase et uniquement une partie supérieure 21. Il peut donc ne pas être au contact du sol dans certains modes de réalisation. Selon un mode de réalisation, le dispositif robotique 1 est fixé au mur par exemple au moyen d'un clip, de vis ou de tout autre moyen de fixation.

Selon un autre exemple, le moyen de déplacement est une feutrine agencée sur la surface inférieure de l'embase 23 de manière à permettre un glissement de cette dernière sur un sol lisse. Selon un autre mode de réalisation, le moyen de déplacement est un coussin d'air propulsé au moyen d'une commande pendant une durée prédéfinie et permettant de déplacer le dispositif robotique 1. Enfin, selon un autre exemple, le moyen de déplacement est un ensemble de microbilles permettant un roulage du dispositif robotique 1 en limitant les frottements.

### Freins

Selon un mode de réalisation, l'embase 23 comprend un frein 26 permettant de bloquer le déplacement de l'embase 23. Le frein 26 permet notamment d'assurer un maintien fixe des roues 25. Il est préférentiellement positionné sur l'embase 23, notamment lorsque le dispositif robotique 1 est déplaçable, par exemple grâce à un moyen de déplacement disposé sur l'embase 23. Le frein 26 permet en outre de ralentir la vitesse de déplacement du dispositif robotique 1 lorsqu'il est actionné pendant son mouvement. Selon un exemple, l'embase 23 comprend une pluralité de freins 26 permettant de stabiliser le dispositif robotique 1. Selon un exemple de réalisation, afin de stabiliser le dispositif robotique 1, l'embase 23 comprend un stabilisateur. Le stabilisateur est activable lorsque le frein 26 est actionné. Il permet, par exemple, d'ancrer l'embase 23 au sol. Ce stabilisateur peut être réalisé par des ventouses, le déploiement d'une masse au contact du sol, un élément de contact avec la surface d'appui tel qu'une béquille ou encore un actionneur permettant de verrouiller le blocage des roues. Selon un mode de réalisation, des plots de stabilisation déployables permettent de maintenir le dispositif robotique 1 de l'invention dans une position fixe.

Lorsque le dispositif robotique 1 est maintenu sur une paroi verticale, telle qu'un mur ou une cloison, le frein 26 peut être agencé sur l'embase 23 ou la partie supérieure 21. Il permet de verrouiller la position du dispositif robotique 1 à une position donnée de la paroi. Le frein 26 peut prendre la forme d'une vis de serrage, un clip permettant de verrouiller la position du dispositif robotique 1 au sein d'une rainure ou d'autres formes.

### Support de fixation

La partie supérieure 21 du bâti 2 comprend un support de fixation d'un bras robotisé 6. La figure 2 représente un exemple d'un bras robotisé 6 de l'invention qui est enfiché dans un support de fixation 24.

Le support de fixation 24 comprend une cavité d'accueil d'une extrémité proximale du bras robotisé 6. Selon différents modes de fixation, le bras robotisé 6 peut être enfiché, clipsé ou vissé dans la cavité d'accueil. Selon différents modes de réalisation, le support de fixation 24 comprend un moyen de serrage et un moyen de verrouillage 241 du bras robotisé 6. Le moyen de serrage permet d'obtenir une solidarité entre le bras robotisé 6 et le bâti 21 en limitant le jeu entre les pièces. Il permet également un meilleur maintien du bras robotisé 6 par le bâti 2. Le moyen de verrouillage 241 permet de sécuriser la fixation afin d'empêcher une désolidarisation accidentelle du bras robotisé 6 du bâti 2.

Le support de fixation 24 est préférentiellement rendu solidaire selon au moins un axe avec le bâti 2. Selon un mode de réalisation, le bras robotisé 6 est fixé sur le bâti 2 selon un axe sensiblement perpendiculaire au plan de la partie supérieure 21. Lorsque le plan principal de la partie supérieure est vertical, le bras robotisé 6 est maintenu selon un axe horizontal au niveau du support de fixation 24.

Le bras robotisé 6 et un bras comprenant une pluralité de portions, chacune étant articulée par rapport à une autre selon au moins un degré de liberté. La portion proximale 60 est la portion destinée à s'emboiter dans le support de fixation 24. La portion distale 65 est la portion destinée à maintenir un dispositif opérateur distal 8. Ce dernier dispositif opérateur distal 8 est destiné à naviguer à proximité de la peau d'un patient pour permettre une interaction, telle qu'une prise de photo, l'émission d'un faisceau laser, un contact sensoriel.

Selon un mode de réalisation, le support de fixation 24 comprend une liaison pivot permettant d'orienter l'axe principal de la cavité d'accueil ou de l'axe proximal du bras robotisé 6, c'est-à-dire de la portion proximale 60 de cette dernière. Selon un mode de réalisation, l'axe de la portion distale 60 du bras robotisé 6 peut donc être agencé perpendiculairement au plan de la partie supérieure 21 lors de son introduction puis il peut être incliné par une action sur le support de fixation 24. A cet effet, le support de fixation 24 peut être rendu mobile selon un degré de liberté. Dans ce cas, il peut par exemple comprendre une poignée d'orientation et un dispositif de verrouillage de l'orientation du bras robotisé 6. Selon un autre cas, le réglage de l'orientation de la base du support de fixation 24 du bras robot 6 est motorisé et contrôlable depuis l'interface ou un bouton. Ainsi, après déverrouillage de l'orientation, un opérateur est en mesure d'orienter le bras robotisé 6 selon une inclinaison souhaitée et de verrouiller la nouvelle orientation de l'axe de la portion distale du bras robotisé 6 maintenue dans le support de fixation 24. Selon un exemple, la liaison pivot est une liaison rotule, par exemple, limitée dans ses degrés de liberté à des portions d'angles. Une telle liaison rotule permet, par exemple, d'orienter l'axe de la portion proximale dans le plan P₁(0, y₁, x₁) ou dans le plan P₂(0, y₁, z₁) du repère R₁ représenté à la figure 1.

Les figures 12 et 13 permettent de représenter deux positions différentes du bras robot 6 qui est fixé au support de fixation 24. Le support de fixation 24 est représenté ici selon un autre exemple. La portion proximale du bras robot 6 a changé d'orientation entre la figure 12 et la figure 13 d'environ 45°. Dans ce cas d'exemple, le support de fixation 24 comprend une liaison pivot permettant d'orienter la partie proximale du bras robot 6 autour d'un axe compris dans le plan horizontal.

### Système d'acquisition d'images

Le dispositif robotique 1 de l'invention comprend une pluralité de dispositifs optiques 3. Selon l'exemple de la figure 1, 6 dispositifs optiques sont agencés sur la partie supérieure du bâti 2. Selon un mode de réalisation, les dispositifs optiques 3 sont disposés dans un même plan et sont par défaut orientés selon un même axe. Un intérêt de l'invention est de configurer un dispositif robotique 1 de l'invention qui puisse offrir un large champ de vue afin d'acquérir des images d'un corps humain en position debout ou allongée.

A cet fin, différentes configurations sont possibles selon différentes variantes de réalisation qui peuvent se combiner entre elles selon le nombre de dispositifs optiques 3 que l'on souhaite mettre en oeuvre.

Une première variante consiste à espacer des dispositifs optiques 3 d'une certaine distance entre eux pour augmenter le champ de vue en largeur Fov₁, dans le plan P₁(0, y₁, x₁) sur la figure 1. Les différents dispositifs optiques 3 sont espacés préférentiellement dans la largeur d'une distance comprise entre 10cm et 2m. Plus spécifiquement, une distance entre deux dispositifs optiques 3 agencés sur un même axe horizontal peut être comprise entre 30 cm et 150 cm. Afin d'obtenir un dispositif robotique 1 le plus compact possible qui puisse, par exemple, être déplacé d'une pièce à une autre, une distance entre deux dispositifs optiques 3 agencés sur un même axe horizontal peut être comprise entre 30 cm et 60 cm. Cette distance entre deux dispositifs optiques 3 permet d'augmenter le champ de vue horizontalement pour acquérir l'image d'un patient ayant les bras décollés du corps s'étendant selon un axe horizontal.

Une seconde variante consiste à espacer des dispositifs optiques d'une certaine distance entre eux pour augmenter le champ de vue en hauteur Fov₂, dans le plan P₂(0, y₁, z₁) sur la figure 1. Les différents dispositifs optiques 3 sont espacés préférentiellement dans la hauteur d'une distance comprise entre 10cm et 2m. Plus spécifiquement, une distance entre deux dispositifs optiques agencés sur un même axe vertical peut être comprise entre 30 cm et 150 cm. Selon une gamme plus petite offrant une meilleure compacité du dispositif robotique 1, la distance séparant deux dispositifs optiques 3 verticalement est comprise entre 30 et 60 cm. Cette distance entre deux dispositifs optiques 3 permet d'augmenter le champ de vue verticalement pour acquérir l'image d'un patient de la tête au pied lorsqu'il est positionné debout.

La figure 12 représente un cas d'exemple dans lequel le dispositif 1 est représenté dans une position donnée où le champ de vue vertical CH₃₁ du dispositif optique 31 est approximativement de 45°.

On nomme un « champ de vue virtuel » une combinaison ou une union de champs de vue réels de plusieurs dispositifs optiques 3. Le champ de vue virtuel correspond à l'association de plusieurs champs de vue réels considérés depuis différentes positions. Ainsi, le champ de vue virtuel est le champ de vue du système optique 3' en considérant une source virtuelle depuis laquelle le champ de vue virtuel pourrait être considéré.

Une troisième variante consiste à positionner des rangées de dispositifs optiques 3 sensiblement alignés selon un axe vertical ou un axe horizontal sur le dispositif robotique 1. Ainsi sur la figure 1, les dispositifs optiques 32, 31 et 35 sont sensiblement alignés verticalement, les dispositifs optiques 33, 334 et 36 sont sensiblement alignés verticalement, les dispositifs optiques 32, 33 sont sensiblement alignés horizontalement, les dispositifs optiques 34, 31 sont sensiblement alignés horizontalement, les dispositifs optiques 36, 35 sont sensiblement alignés horizontalement.

Cette configuration permet d'offrir une couverture d'acquisition permettant d'acquérir des images d'un patient debout ou allongé sur une table tout en offrant un encombrement réduit.

Selon un exemple de réalisation, plusieurs dispositifs robotiques 1 de l'invention peuvent être combinés pour acquérir un champ de vue plus important ou pour obtenir un scan complet d'un patient dont sa face avant et sa face arrière sans qu'il n'ait besoin de se retourner. Une calibration entre les dispositifs robotiques 1 est alors réalisée pour représenter les nuages de points générés à partir des images acquises dans un même référentiel.

La figure 6 représente un exemple de positionnement d'un patient 100 se tenant debout devant le dispositif robotique 1 dont uniquement le système optique 3' est représenté. Dans cette figure, les champs de vue virtuels en largeur et en hauteur FoV₁ et FoV₂ du système optique 3' sont représentés. Les champs de vue virtuels sont construits par association des champs de vue réels de chaque dispositif optique 31, 32, 33, 34, 35, 36. La répartition sur une surface donnée permet d'élargir le champ de vue virtuel du système optique 3' afin de couvrir la surface entière du corps d'un humain 100. Un intérêt d'un agencement d'une pluralité de dispositifs optiques 3 distancés chacun d'eux d'une distance donnée est de permettre d'augmenter le champ de vue global du système optique 3' tout en conservant des optiques avec une profondeur de champ permettant de générer une carte de profondeur suffisamment précise.

Un autre avantage est de réduire l'occlusion causée par les déplacements du bas robot 6 devant le système optique 3'. Ainsi, en multipliant les dispositifs optiques 3, la génération du nuage de points global, à partir de différents nuages de points générés depuis différents points de vue, peut compenser les effets d'occlusion et de masquage du corps du patient 100 par le bras robot 6.

### Dispositif optique

Chaque dispositif optique 3 est configuré pour générer une image 3D. Dans le cadre de la présente invention, une image 3D peut s'entendre comme un ensemble d'images d'un même sujet, au moins deux images, permettant de générer une information de profondeur dudit sujet. Selon un exemple, l'image 3D peut être une image stéréoscopique, c'est-à-dire une paire d'images, telle que deux vues dites gauche et droite, réalisée par deux capteurs optiques prenant une même scène à partir de deux points de vue légèrement distants. Le spectre d'une telle image peut être dans le visible ou dans l'infrarouge. Dans ce cas, une image 3D peut être une paire d'images couleurs ou infrarouges. Selon un autre exemple, une image 3D peut également s'entendre comme une image qui est directement associée à une carte de profondeur, selon un autre exemple, une image 3D peut s'entendre comme un nuage de points représentant un échantillonnage d'une surface tridimensionnelle obtenue à partir d'images acquises d'un sujet. L'image 3D peut également comprendre d'autres informations, en plus des points, telles que des informations de couleurs. Selon un mode de réalisation, l'image 3D comprend une image 2D et une information de profondeur estimée par un algorithme d'estimation de profondeur. Selon un mode de réalisation, l'image 3D comprend une image 2D et un maillage tridimensionnel calculée selon le procédé de l'invention.

Plus généralement, on nomme une image 3D : une image 2D à laquelle est combinée une information numérique permettant de construire une représentation tridimensionnelle d'une surface dans l'espace. Cette information numérique peut être une seconde image, une carte de profondeur ou tout autre donnée calculée permettant construire un nuage de points dans l'espace à partir d'une image 2D.

Selon un mode de réalisation, chaque dispositif optique 3 comprend une paire de caméras permettant de générer une carte de profondeur de l'image acquise. Selon un exemple de réalisation, les caméras sont des caméras infrarouges. Les caméras infrarouges offrent des résolutions permettant de dresser une carte de profondeur dont la précision permet de restituer des différences de reliefs de la surface d'un corps d'un patient situé à une distance entre 30cm et 3m, voire 4m, du dispositif robotique 1.

Selon une méthode non revendiquée, des images 2D acquises d'un même objet depuis deux points de vue dont on connait l'agencement peuvent être combinées selon des techniques connues pour obtenir une carte dite de profondeur ou de distance.

Selon un exemple de réalisation, chaque dispositif optique 3 comprend 3 caméras dont une paire de caméras infrarouges et une caméra couleur.

Selon un exemple, les dispositifs d'acquisition peuvent comprendre une paire de caméras infrarouge 3D couleur et au moins un projecteur infrarouge. Selon un mode de réalisation, le dispositif optique 3 comprend un projecteur infrarouge laser permettant de projeter une image comportant des motifs, également appelé « patterns », notamment à la surface d'un corps humain. Le motif projeté est alors acquis par la paire de caméras infrarouges afin de reconstruire en temps réel par stéréoscopie une carte de profondeur.

Selon un autre exemple, les dispositifs d'acquisition peuvent comprendre une paire de caméras couleur configurées pour faire de la stéréo passives afin de générer les images 3D.

Selon un autre exemple, les dispositifs d'acquisition peuvent comprendre une caméra couleur et un projecteur de lumière projetant des motifs de lumière structurée.

Les images acquises peuvent alors être traitées de sorte à analyser la déformation du motif projeté. Cette technique permet d'obtenir une donnée supplémentaire et permet d'améliorer la reconstruction 3D. En particulier, la détection d'une déformation d'un motif projeté permet d'améliorer la précision de la carte de disparité entre deux images utilisées pour générer la carte de profondeur. En outre, le projecteur permet de réduire le bruit lors de la construction du nuage de points pour générer le premier graphe G₁.

Selon un mode de réalisation, les motifs de l'image sont des motifs réguliers par exemple représentant une forme régulière. Selon un autre mode de réalisation, les motifs sont générés de manière aléatoire.

Selon des variantes de réalisation, d'autres technologies peuvent être mises en oeuvre dans l'invention pour reconstruire une carte de profondeur par exemple à partir de caméra temps de vol. Dans ce dernier cas, la caméra 3D peut être, par exemple, remplacée par une caméra temps de vol, plus connu sous l'appellation anglo-saxonne « Time-of-Flight », ToF. Dans ce cas, il s'agit d'un rayon laser ou lumineux visible ou infrarouge couplé à une caméra ou un récepteur photosensible permettant de mesurer le temps de vol de différents rayons tirés dans l'espace et donc d'en reconstruire une cartographie 3D.

D'autres techniques de construction d'une carte de profondeur peuvent être utilisées, telles qu'un dispositif émetteur-récepteur laser. La réflexion du faisceau laser permet de générer une donnée relative à la géométrie de la surface de réflexion. Une autre technique peut s'appuyer sur un émetteur-récepteur à ultrasons. Un exemple d'un système qui pourrait être mis en oeuvre est le système de type LIDAR.

### - Calibration

Une calibration, dite « calibration stéréoscopique », inter-caméras peut être réalisée par exemple entre les deux caméras d'un même dispositif optique 3 pour réaliser une reconstruction 3D d'un sujet. La calibration vise à calculer une transformation entre deux images acquises par chacune des caméras. Cette calibration permet d'assurer les transformations entre les repères des différentes caméras/projecteurs d'un même système optique 3', par exemple, les transformations entre les 3 caméras/projecteur 331, 332, 333 représentés à la figure 10 du dispositif optique 33.

Enfin, cette calibration permet d'assurer les transformations entre un dispositif optique 3 et un autre dispositif optique 3. La calibration peut comprendre la définition d'une caméra de référence d'un dispositif optique 3 afin que les transformations s'effectuent par chaîne vers n'importe quel repère du dispositif dans lequel la caméra de référence est définie.

Une calibration du bras robot 6 permet de connaitre la transformation entre le repère commun de tous les dispositifs optiques, par exemple choisi arbitrairement parmi l'ensemble des repères de chaque dispositif optique 3, et celui du bras robot 6 qui peut être défini au niveau de l'origine du bras.

Les repères suivants sont définis :R₀ : référentiel monde dans lequel évolue le dispositif robotique 1
R₁ : référentiel lié au dispositif robotique 1 en un point du bâti ;
R₂ : référentiel lié à la partie fixe du bras robot 6 vis à vis du bâti du dispositif robotique 1 ;
R₃ : référentiel lié à la tête du bras robot 6 ;
R₃₁ : référentiel lié au dispositif optique 31 ;
R₃₂ : référentiel lié au dispositif optique 32 ;
R₃₃ : référentiel lié au dispositif optique 33 ;
R₃₄ : référentiel lié au dispositif optique 34 ;
R₃₅ : référentiel lié au dispositif optique 35,
R₃₆ : référentiel lié au dispositif optique 36.

Le procédé de l'invention permet donc de calibrer le bras robotisé 6 par rapport au système d'acquisition 3' ou au bâti 2 et donc de réaliser les transformations des images dans les référentiels R₃->R₁. Lorsqu'un référentiel R₃ est associé à la tête 8 du bras robotisé 6, une transformation de R₃ -> R₁ est connue du fait que la cinématique d'articulation de chaque partie du bras robot 6 est connue par un calculateur et une mémoire du système pilotant ledit bras robot 6.

Selon un exemple, un dispositif optique 3 est déterminé comme une référence. Un avantage est, d'une part, de calibrer toutes les caméras du système optique 3' vis-à-vis d'un seul dispositif optique 3 et, d'autre part, de calibrer la position du bras robot 6 vis-à-vis de ce même dispositif optique 3. Ainsi, cette calibration vis-à-vis d'une unique caméra ou d'un unique dispositif optique 3 de référence permet d'assurer que les déplacements du bras robot 6 sont bien référencés dans un repère commun au système optique 3'.

Le calcul des transformations peut être aussi effectué par un processus d'alignement itératif des différents nuages de points générés par les différents systèmes optiques. Un tel algorithme est appelé "itérative closest points" dans la terminologie anglo-saxonne.

Selon un autre mode de réalisation, la calibration peut également être effectuée en étudiant la transformation qu'il y'a entre une paire d'image couleur.

La calibration peut être réalisée à partir d'un motif de référence. Typiquement, une projection d'une image peut être utilisée. Il peut s'agir d'une projection d'un motif par le projecteur associé à un dispositif optique 3. Un exemple de réalisation comprend la projection d'un damier dont les déformations acquises par chaque caméra permettent de générer un facteur correctif pour compenser la position ou l'orientation de ladite caméra vis-à-vis d'une autre caméra, ou respectivement d'un dispositif optique 3 vis-à-vis d'un autre dispositif optique 3.

Cette calibration assure qu'un nuage de points calculés par un dispositif optique 3 corrobore avec un autre nuage de points calculés par un autre dispositif optique 3.

### - Orientable

Selon un mode de réalisation, au moins un dispositif optique 3 est orientable selon un axe A-A' représenté sur la figure 10. Le pivotement du dispositif optique 3 permet d'obtenir des configurations de champ de vue modulable. A titre d'exemple, une orientation d'un ou plusieurs dispositifs optiques 3 permet d'adapter un champ de vue par exemple à un cas spécifique tel qu'un patient de petite taille ou d'adapter la prise de vue à une position d'un patient couché ou debout. Le pivotement peut être configuré dans une gamme d'angles de [-60°; +60°] vis-à-vis d'un axe par défaut compris dans le plan horizontal. Selon un mode de réalisation, au moins deux degrés de liberté peuvent être contrôlés pour orienter chaque dispositif optique ou au moins une partie des dispositifs optiques. Selon un mode de réalisation, 3 rotations par liaison rotule sont pilotables.

Les figures 12 et 13 représentent le dispositif optiques 31 selon deux orientations différentes qui sont adaptées à la position du patient 100. Dans la figure 12, le dispositif optique 31 est orienté pour couvrir une portion du buste d'un patient en position debout. Dans la figure 13, le dispositif optique 31 est orienté pour couvrir une portion du buste d'un patient en position allongée. Le pivotement entre les deux orientations du dispositif 31, c'est-à-dire entre les deux droites A₃₁ et A₃₁' peut correspondre à un angle de +20°/ axe horizontal dans la figure 12 et de -40° / axe horizontal dans la figure 13.

Selon un exemple de réalisation, le ou les pivotement(s) d'un ou plusieurs dispositifs optiques 3 peut(vent) être piloté(s) depuis une console ou d'une interface utilisateur dudit dispositif robotique 1.

Selon un exemple, au moins un dispositif optique 3 est amovible et peut être remplacé par un dispositif optique 3 différent. Un intérêt est de changer le champ de vue du dispositif optique 3.

Selon un autre exemple, le dispositif optique 3 est amovible pour être clipsé selon une autre position. Ainsi, dans cette configuration chaque dispositif optique 3 peut être inséré dans un logement permettant d'offrir des orientations différentes.

Selon un exemple de réalisation, afin d'ajuster le champ de vue du système d'acquisition 3' à un positionnement d'un patient, le bâti 2 peut comprendre une partie mobile par exemple en rotation. La figure 9 représente un exemple de liaison pivot agencée entre le bâti 2 et l'embase 23 qui permet de faire pivoter le bâti 2 d'un angle donné.

Selon un autre exemple, des ailettes 22 peuvent être rendues orientables, soit selon un axe de rotation vertical soit, selon un axe de rotation horizontal.

### Dispositif électronique

Le dispositif robotique 1 de l'invention comprend un dispositif électronique (non représenté) comprenant une entité de calcul qui comprend à un minima un calculateur qui peut être un composant électronique muni d'un processeur. Le dispositif électronique comprend, en outre, une mémoire pour stocker des paramètres de configurations du dispositif robotique 1, les paramètres de calibration du dispositif robotique 1 et notamment des optiques du système optique 3', les instructions nécessaires à l'exécution les logiciels fonctionnant pour calculer les trajectoires du bras robot, les surfaces de corps d'un patient, les données de configuration et de paramétrage des capteurs, des émetteurs ou de tout autre équipement piloté par le bras robot 6, des données telles que celles permettant d'exécuter le réseau de neurones utilisés pour reconstruire la surface du corps d'un patient à partir de données optiques comme une image, une carte de profondeur ou un nuage de points. Ces dernières données du réseau de neurones peuvent comprendre les coefficients du réseau, des données d'un classificateur, des données d'une fonction de régression.

Le dispositif électronique peut être un unique composant ou encore dans une seconde réalisation, il peut comprendre différents composants qui sont associés à différents éléments du dispositif robotique 1, tels que le bras robot 6, le système optique 3', l'écran 4.

Un premier objectif du dispositif électronique est de générer une surface d'un corps d'un patient en temps réel à partir des images acquises par le système optique 3'.

### - Nuage de points

A partir de la carte de profondeur, l'invention permet de générer un nuage de points dans l'espace représentant un maillage de la surface du corps d'un humain. Chaque point du nuage de points peut être associé à une coordonnée spatiale tridimensionnelle d'un référentiel lié au dispositif robotique 1. Les points constituent alors un premier graphe G₁. Selon un exemple, les points du premier graphe G₁ obtenus sont non orientés et non connectés. Dans sa forme la plus simple, le graphe G₁ est une liste de points non ordonnée dont les coordonnées sont référencées dans l'espace, par exemple dans un référentiel de l'espace dans lequel est situé le dispositif robotique 1 ou un référentiel associé au dispositif robotique 1. Les points sont alors définis chacun indépendamment des autres. Dans sa forme la plus simple, le graphe G₁ est donc un nuage de points.

Selon un mode de réalisation, le procédé de l'invention permet de récupérer un nuage de points issu des images acquises et de le transférer par exemple sur un processeur graphique tel qu'un GPU, GPU désignant dans la terminologie anglo-saxonne « Graphic Processing Unit ».

Les images peuvent être traitées de sorte à extraire un périmètre d'acquisition limitant la zone à traiter. Pour cela, un gabarit ou un masque de segmentation, éventuellement obtenu grâce à un réseau de neurone, peut être utilisé pour éviter de prendre en considération des points en dehors d'une zone d'intérêt défini par la surface du corps.

L'acquisition des images et la couverture de l'intégralité de la surface à traiter permettent de générer une carte de profondeur de la totalité du corps d'un patient. La carte de profondeur comprend une grille de points/de pixels dont l'intensité correspond à la distance relative avec la caméra.

Selon un autre exemple, aucune surface n'est traitée à ce stade du procédé. Les distances entre points sont générées indépendamment d'une reconnaissance de forme telle qu'une enveloppe d'un corps.

Selon un exemple, le nombre de points générés par unité de surface est configurable selon une résolution souhaitée du nuage de points. L'acquisition peut être configurée pour augmenter la résolution spatiale des nuages de points en augmentant la résolution des images. Elle peut aussi être configurée pour augmenter la résolution temporelle en augmentant le nombre d'images collectées par unité de temps.

Selon un mode de réalisation, chaque dispositif optique 3 calcule un nuage de points à partir des images acquises. Chaque dispositif optique 3 comprend un ensemble de points. Ainsi selon l'exemple de la figure 1 à chaque acquisition, le système optique 3' active l'ensemble des dispositifs optiques 3 de sorte que :
- le dispositif optique 31 génère un ensemble ENS₃₁ de points du nuage de points ;
- le dispositif optique 32 génère un ensemble ENS₃₂ de points du nuage de points ;
- le dispositif optique 33 génère un ensemble ENS₃₃ de points du nuage de points ;
- le dispositif optique 34 génère un ensemble ENS₃₄ de points du nuage de points ;
- le dispositif optique 35 génère un ensemble ENS₃₅ de points du nuage de points,
- le dispositif optique 36 génère un ensemble ENS₃₆ de points du nuage de points.

Le dispositif électronique comprend des moyens de calculs pour générer un nuage de points global, noté ENS_{G}, comprenant l'ensemble des points des différents ensembles générés ENS₃₁, ENS₃₂, ENS₃₃, ENS₃₄, ENS₃₅, ENS₃₆. Un intérêt est d'obtenir un nuage de points le plus complet possible représentatif du corps d'un patient en gardant un système compact.

Le dispositif électronique permet de réaliser différentes opérations suivantes :
- application d'une fonction de transfert basée sur une configuration de calibration des différents dispositifs optiques 3 vis-à-vis d'un point de référence du dispositif robotique 1 ;
- corrélation entre les différents ensembles de points afin de déterminer un sous-ensemble de points en commun et un sous-ensemble de points non partagés par les différents nuages de points ;
- optionnellement, contrôle d'erreurs de calibration d'un dispositif optique 3 ;
- détermination d'un nuage de point global comprenant l'ensemble des points des différents ensembles de points générés par chaque dispositif optique 3.
- **Traitement du nuage de points et calcul des descripteurs de forme**

Le dispositif robotique 1 de l'invention comprend un calculateur pour reconstituer la surface d'un corps humain fidèlement à celle d'un patient à partir des images acquises pour calculer des descripteurs de forme locaux. Selon un mode de réalisation, un graphe connecté est obtenu à partir du maillage de points. La méthode consiste à associer à chaque point du graphe connecté des attributs issus de calculs effectués sur la matrice adjacente du graphe connecté. Cela correspond au calcul de fonctions appliquées à chaque point, dont ses coordonnées, et leur voisinage de manière à générer des descripteurs de forme, ou coefficient de forme.

Un avantage des descripteurs de forme est de caractériser une topologie locale de la surface à un point du premier graphe G₁. Lorsque le corps d'un patient bouge ou change au cours du temps, la surface du corps est considérée comme un objet déformable dont les transformations sont non isométriques. Les descripteurs de forme permettent de générer en chaque point des caractéristiques propres qu'il est possible de retrouver grâce au procédé de l'invention après une déformation.

### - Utilisation d'un réseau de neurones entrainé

Le dispositif de l'invention comprend un composant électronique comprenant au moins une mémoire et un calculateur pour calculer un modèle de corps humain à partir d'un réseau de neurones entrainé. L'implémentation du réseau de neurones entrainé peut être réalisée de sorte qu'il traite, en entrée, des vecteurs comportant les coordonnées de chaque point du nuage de points et des attributs précédemment calculés associés auxdits points et, en sortie le réseau de neurones produit un nouveau nuage de points connecté, tel qu'un graph connecté, qui représente la surface du corps du patient dont les images ont été acquises. La surface du corps générée est également appelée un modèle de corps paramétré MOD_P dans la mesure où le paramétrage correspond à celui du patient et qu'il est généré à partir des images acquises. Selon un autre mode de réalisation, le modèle de corps est non paramétrique.

Selon un exemple de réalisation, l'entrainement d'un tel réseau peut être réalisé à partir d'un modèle paramétrique de corps humain défini par des paramètres tels que des paramètres de formes et de rotations d'articulations.

Alternativement, en sortie du réseau de neurones entrainé, il peut être calculé par une régression des paramètres d'un modèle paramétrique de corps humain pour générer un modèle de corps correspondant à celui du patient. Le dispositif électronique est alors en mesure de calculer le nuage de points correspondant au modèle paramétrique paramétré avec les paramètres issus de la régression du réseau de neurones.

Une autre alternative est d'utiliser un réseau de neurones entrainé permettant de générer directement un graphe G connecté correspondant au nuage de point mesuré du patient. Cette solution permet de ne pas utiliser de modèle paramétrique de corps en sortie.

Ainsi, la surface d'un corps humain peut être calculée en temps réel à partir d'une acquisition d'images par le dispositif robotique 1 et un réseau de neurones entrainé.

Un intérêt d'un calcul en temps réel de la surface modélisée du corps d'un patient est d'anticiper, par exemple, des mouvements dudit patient de prendre en compte des mouvements, par exemple, issus de la respiration du patient 1.

L'avantage du procédé de l'invention est de pouvoir générer une nouvelle surface modélisée en moins de 30ms. Ces temps de réponse permettent notamment d'asservir les mouvements d'un bras robot 6 avec une vitesse suffisante pour anticiper des collisions ou des mouvements brusques afin de se dégager le plus rapidement possible. La configuration d'une acquisition par une caméra peut être par exemple de 10 à 90 fps.

Selon un mode de réalisation, une première image tridimensionnelle peut être générée par le dispositif électronique 3 partir de l'ensemble des images acquises par le système optique 3' de sorte à obtenir une vue globale du corps d'un patient.

### - Guidage du bras robot

Un second objectif du dispositif électronique est de générer des consignes de guidage en temps réel pour asservir les mouvements du bras robot 6 selon une trajectoire réactualisée en fonction, d'une part, d'une stratégie de planification prédéfinie et, d'autre part, des données recalculées en temps réel correspondant à la surface du corps MOD_P du patient. Les données du corps du patient MOD_P sont réactualisées en temps réel, car le patient peut possiblement bouger accidentellement ou changer de position à la demande d'un médecin, ou respirer pendant que le bras du robot est en mouvement sur à proximité de la cage thoracique, etc.

Le bras robot 6 est guidé par un contrôleur de mouvement GEN_TRAJ qui prend en compte différentes contraintes pour asservir le bras robot 6. Notamment, le contrôleur CTRL peut prendre en compte une consigne provenant d'un module de détection de collision pour adapter la course du bras robot en temps réel. Enfin, le contrôleur CTRL prend en considération des données provenant de la tête du robot 6 au moyen d'un module CNTR_8, par exemple lorsqu'une détection d'une image singulière impose une manipulation particulière du robot, telle qu'une modification de la course, un arrêt de la course, etc.

Le guidage d'un bras robot 6 est effectué à partir d'une trajectoire de traitement, notée 30 sur la figure 11, établie au sein d'une zone définie 9 de la surface ainsi modélisée et recalculée en temps réel.

La trajectoire 30 peut être obtenue à partir d'une stratégie de balayage du bras robot 6. Selon un exemple, la trajectoire générée 30 est obtenue grâce à un module de planification PLAN_TRAJ qui accessible par exemple à partir d'une interface utilisateur 4 ou de toute autre interface utilisateur déportée du dispositif robotique 1.

A partir de la trajectoire 30, des trajectoires associées peuvent être calculées pour guider le bras robot 6. Il peut s'agir de la trajectoire du dispositif opérateur distal 8 par exemple lorsqu'une certaine distance entre le dispositif opérateur distal 8 et la surface de la peau d'un patient doit être respectée.

Dans l'exemple de la figure 11, trois singularités 91 dermatologiques sont représentées sur une portion 9 de peau d'un patient 1. Il peut s'agir par exemple d'une cicatrice, d'un mélanome, d'un grain de beauté, ou de toute autre singularité de la surface de la peau. Lorsque le champ optique 87 du dispositif opérateur distal 8 intercepte une singularité 91, le bras robot 6 peut être configuré pour arrêter sa course ou activer un autre équipement tel qu'une optique pour acquérir des images des singularités qui sont analysées dynamiquement en temps réel pour asservir le contrôle du bras robot 6 ou à postériori pour établir un diagnostic par exemple.

Selon un exemple, l'orientation entre l'axe du dispositif opérateur distal 8 et la surface de la peau est déterminée par défaut à 90°. Cet angle correspond à la situation où l'axe principal d'un capteur ou d'un émetteur du dispositif opérateur distal 8 du bras robot 6 est confondu avec la normale N au point cible 35 de la surface du corps considéré. La figure 5 représente une vue dans laquelle la partie distale 65 du bras robot 6 est illustrée au-dessus d'un patient allongé.

L'extrémité du dispositif opérateur distal 8 est positionnée à une distance donnée du point de visé 35 situé à la surface du corps modélisé situé, par exemple, sur la trajectoire 30 générée.

Selon une autre réalisation, l'extrémité peut également être appliquée contre la peau du patient. Le contrôleur est configuré pour réguler la force appliquée sur la peau du patient pour, par exemple, poser un Dermatoscope, ou une sonde ultrason.

Le dispositif robotique 1 de l'invention comporte, en outre, un calcul d'une nouvelle cinématique du bras robot 6 asservie sur la définition de la nouvelle trajectoire 30. Un avantage est d'obtenir de bonnes performances de guidage en temps réel. La prise en compte d'un modèle de corps simplifie les calculs de guidage. L'asservissement du bras robot 6 peut être rapide et ainsi il permet de limiter les cas de collisions ou les mouvements brusques d'un patient.

Le dispositif opérateur distal 8 peut être liée à un repère noté R₃ (O₃, x₃, y₃, z₃) représenté à la figure 4. La position du dispositif opérateur distal 8 du bras robot 6 peut être facilement identifiée dans le repère R₂ (O₂, x₂, y₂, z₂) avec la connaissance de la cinématique animant les différentes parties du bras robot 6. Un intérêt est de référencer les images acquises par le dispositif opérateur distal 8 par exemple dans le repère R₁ (O, x₁, y₁, z₁) ou R₀ (O, x₁, y₀, z₀).

Ainsi, le dispositif électronique du dispositif robotique 1 est configuré pour délivrer des consignes de guidage en temps réel à l'élément moteur du bras robot qu'il soit en mesure de déterminer la cinématique de chaque partie du bras robot 6.

### Bras robot

Selon un mode de réalisation, le bras robot 6 comprend un ensemble de branches 61, 62, 63, 64, 65 articulées au moyen d'articulations pivots 612, 623, 634, 645, 656. Le bras robot 6 comprend dans ce cas 5 branches articulées à partir de liaisons pivots. Selon une variante, il comprend 6 branches articulées et selon d'autres variantes, il peut comprendre 7, 8 ou 9 branches articulées. En effet, dans ce mode de réalisation, chaque articulation est capable d'effectuer au moins une rotation entre deux branches adjacentes.

Selon un mode de réalisation, chaque branche du bras robot 6 comporte un capteur d'effort. Un avantage est de prévenir d'une collision brusque. Lorsqu'un contact est établi entre une branche du bras robot 6 et un objet, le mouvement du bras robot peut être automatiquement arrêté ou son guidage modifié de manière à ce qu'il change sa trajectoire. Un autre avantage est de calculer l'effort appliqué sur le patient lorsque l'opérateur distal est en contact avec sa peau.

Selon un mode de réalisation, une étape de calibration du bras robot 6 est réalisée préalablement au procédé de l'invention. La calibration peut comprendre les positionnements relatifs du bras robot 6 dans le repère R₂ vis-à-vis du repère R₁ ou de n'importe quel repère R₃₁, R₃₂, R₃₃, R₃₄, R₃₅, R₃₆ associé à un dispositif optique 31, 32, 33, 34, 35, 36 du système optique 3' ainsi que sa position initiale.

### Dispositif opérateur distal

Selon un mode de réalisation, le bras robot 6 comporte un dispositif opérateur distal 8 avantageusement agencé à l'extrémité du bras robot 6. Selon un exemple, le dispositif opérateur distal 8 comprend au moins un capteur et/ou au moins un émetteur. Le dispositif opérateur distal 8 est également appelée la « tête » du bras robot 6.

Selon les modes de réalisation, un système optique distal 83, 84 peut être agencée à l'extrémité de cette du dispositif opérateur distal comme dans le cas de la figure 7. Un intérêt de la configuration de la figure 7 est de configurer un bras robot 6 pour des applications dermatologiques de prises d'images.

Dans l'exemple de la figure 7, le bras robotisé 6 est muni d'un ou plusieurs capteurs optiques 67 pour acquérir des images locales de la surface de la peau. Un intérêt de la prise d'images locales en tête du bras robot 6 est la possibilité de reconstruire une surface du corps « colorisé », c'est-à-dire dans laquelle les images prises sont intégrées au modèle de corps paramétré MOD_P généré par le procédé de l'invention. A cette fin, le dispositif optique 3 situé à l'extrémité du bras robot 83, 84 peut comprendre préférentiellement deux optiques de sorte à reconstituer une carte de profondeur associée aux images acquises simultanément par les deux optiques. Les images peuvent alors être indexées sur la surface du corps paramétré. On comprend, par exemple, que les images des singularités 91 de la figure 11 peuvent être enregistrées et associées à une position d'un modèle de corps MOD_P d'un patient. Ainsi, plusieurs mois plus tard, lors d'un contrôle, le dispositif robotique 1 est capable de générer une trajectoire 39 passant par un ensemble de points associés à des singularités pour effectuer un contrôle de l'évolution des images des singularités vis-à-vis d'images de référence.

Selon un mode de réalisation, le dispositif opérateur distal 8 comprend deux optiques locales 83, 84 pour prendre des images de la surface du corps et une optique millimétrique 85 ou microscopique amovible bidirectionnelle selon une direction longitudinale 86. Un intérêt est de générer des images avec une résolution millimétrique comportant une résolution pour des applications dermatologiques, par exemple pour des applications de diagnostic.

Le second dispositif optique distal 85 permet donc de prendre une image d'une zone rapprochée de la surface de la peau d'un patient 1. Selon un exemple, le second dispositif optique distal 85 est un Dermatoscope. Le second dispositif optique distal 85 peut comprendre une plaque en verre venant au contact de la peau. Selon un exemple de réalisation, le second dispositif optique distal 85 peut comprendre un émetteur de lumière polarisée de manière à obtenir une information d'image en profondeur de l'épiderme.

A cet effet, la dernière branche 65 du bras robot 6 ou le dispositif opérateur distal 8 peut/peuvent être équipé(s) d'un capteur de retour d'effort afin de freiner sa course du second dispositif optique distal 85, voire l'arrêter et la stabiliser. Lorsque le second dispositif optique distal 85 est en contact de la peau d'un patient 1, le déclenchement d'une prise de vue peut être engagé.

Selon un autre exemple, chaque axe du bras robot 6 est équipé d'un capteur d'effort. L'analyse des valeurs d'effort relevées sur les capteurs est utilisée pour asservir la pression exercée sur la surface. Ainsi, le bras robot 6 grâce à ses capteurs de couple dans chaque axe permet d'asservir une pression de contact entre un outil et la surface de travail, dans ce cas la peau d'un patient. Cela peut être utilisé également pour effectuer un « glissement » de l'outil sur une surface de travail.

Selon un mode de réalisation, un dispositif de balayage 89 est activé afin d'écarter les poils de l'optique 85. Un tel dispositif de balayage 89 peut être automatiquement mis en oeuvre lors de la stabilisation de l'optique du second dispositif optique distal 85. Un intérêt est d'éviter la prise de vue de poils situés à proximité d'une singularité dont on souhaite réaliser une prise de vue. Le dispositif de balayage 89, selon un mode de réalisation, comprend une tige en rotation qui active des pinceaux flexibles à proximité de l'optique 85 ou un système pneumatique. Un avantage est de faire tourner le poil de sorte qu'il ne reste pas entre l'optique 85 et la peau. Selon un exemple de réalisation, une pluralité de dispositifs de balayage 89 est agencée autour de l'optique 85. La figure 6B en représente deux.

Lorsque la seconde optique 85 a capturé une ou plusieurs images avec une résolution plus précise de la singularité, le second dispositif 85 peut effectuer un mouvement de rétractation pour revenir à sa position initiale. Le bras robot 6 peut alors continuer sa course et reprendre la trajectoire de guidage qui a été planifiée.

Selon un exemple, de réalisation, le guidage est réalisé à une distance comprise entre 1cm et 20 cm de la surface du corps d'un patient 1. Selon un mode préférentiel, le guidage est réalisé à une distance comprise entre 2cm et 8 cm de la surface de la peau.

En outre, le dispositif opérateur distal 8 peut comprendre un ou plusieurs émetteur(s) de type ultrasons, radiofréquences ou laser ou toute autre source de signaux susceptibles d'être utilisés pour générer un faisceau incident. Selon un mode de réalisation, le dispositif opérateur distal 8 peut comprendre un réseau d'émetteurs focalisés et des moyens de contrôle de la direction et de la puissance émise.

Selon une autre configuration, le dispositif opérateur distal 8 comprend un dispositif laser. Ce mode de réalisation peut également être compatible de la présence d'une optique non représentée sur la figure 8.

Lorsqu'un émetteur ultrason est agencé à l'extrémité du dispositif opérateur distal 8, un récepteur ultrason peut être associé pour mesurer le signal réfléchi de manière à construire une image du derme et/ou une image de l'épaisseur de la peau d'un patient, voire une image de vaisseaux ou d'un organe.

### Ecran

Selon un mode de réalisation, le dispositif robotique 1 comprend un écran 4 permettant d'afficher le modèle du corps MOD_P du patient reconstruit à partir des images acquises par le système d'acquisition 3'. Ainsi, un opérateur peut visualiser le modèle tridimensionnel du patient et sa position dans l'espace. Cette visualisation permet à un opérateur de prérégler le dispositif robotique 1 de l'invention, par exemple les orientations des dispositifs optiques 3 ou encore la position du dispositif robotique 1 ou encore une position par défaut du bras robot 6.

Selon un mode de réalisation, l'écran 4 est tactile. Il peut comprendre la génération de boutons numériques permettant de commander ou configurer le dispositif robotique 1 ou du bras robot 6. Selon un exemple, un bouton d'arrêt est accessible. Selon une autre réalisation, des actionneurs mécaniques ou optiques peuvent être alternativement agencés sur le dispositif robotique 1 de l'invention afin qu'ils soient directement accessibles sans avoir à interagir avec l'écran 4. Selon un exemple, de réalisation un bouton d'arrêt d'urgence est positionné sur le dispositif robotique 1 afin d'arrêter les mouvements du bras robot 6.

Selon un exemple de réalisation, un mouvement sur le bras robot entraine son arrêt. Selon cette configuration, le bras robot 6 comprend un capteur haptique afin de discriminer une pression ou une force appliquée sur le corps du bras robot 6. Lorsqu'un seuil d'effort est mesuré, le bras robot 6 s'immobilise immédiatement avant de reprendre sa course à partir d'une nouvelle commande de démarrage ou de reprise reçue depuis l'interface utilisateur.

## Revendications

1. Dispositif robotique (1) assurant le guidage automatique d'un bras robotisé (6), comprenant un bâti (2) maintenant un bras robotisé (6) articulé selon une pluralité d'axes de liberté, ledit bras robotisé (6) comportant un dispositif opérateur distal (8) pour générer une interaction homme-machine, ledit dispositif robotique (1) comportant, en outre, un système d'acquisition d'images (3') comportant aux moins deux dispositifs optiques (3, 31, 32, 33, 34, 35, 36) solidaires dudit bâti (2) et agencés en au moins deux positions distinctes du bâti (2), chaque dispositif optique (3) étant configuré pour acquérir une image 3D, le système d'acquisition d'images (3') étant configuré pour acquérir une pluralité d'images 3D d'un corps humain d'un patient provenant d'au moins des deux dispositifs optiques (3), le dispositif robotique (1) comportant une unité de calcul pour générer en temps réel un modèle de corps humain (MOD_S) dudit patient à partir desdites images 3D acquises et une trajectoire de guidage (TRAJ) référencée à la surface dudit modèle de corps humain (MOD_S), les mouvements dudit bras robotisé (6) étant asservis sur ladite trajectoire de guidage (TRAJ).

2. Dispositif robotique (1) selon la revendication 1, **caractérisé en ce que** :
▪ au moins un premier dispositif optique (31, 32, 33, 34) comprend un premier agencement linéaire d'au moins deux caméras selon un premier axe (A₁) ;
▪ au moins un second dispositif optique (35, 36) comprend un second agencement linéaire d'au moins deux caméras selon un second axe (A₂).

3. Dispositif robotique (1) selon la revendication 1, **caractérisé en ce qu'**au moins un dispositif optique (3) comprend un moyen d'orientation de l'axe d'acquisition (N) par l'actionnement d'une liaison mécanique.

4. Dispositif robotique (1) selon la revendication 1, **caractérisé en ce que** chaque dispositif optique (31, 32, 33, 34, 35, 36) comprend :
▪ au moins deux caméras infrarouges et/ou ;
▪ au moins une caméra couleur et/ou ;
▪ au moins une paire de caméras couleur configurées pour générer les images 3D par stéréoscopie et/ou ;
▪ au moins trois caméras comprenant deux caméras infrarouges et une caméra couleur et/ou ;
▪ au moins un projecteur infrarouge projetant un motif et deux caméras infrarouges capturant ledit motif projeté à la surface d'un corps humain.
▪ au moins un projecteur de lumière projetant des motifs de lumière structurée et au moins une caméra capturant lesdits motifs et/ou;
▪ une caméra temps de vol et/ou ;
▪ un dispositif émetteur-récepteur laser.

5. Dispositif robotique (1) selon la revendication 1, **caractérisé en ce que** chaque dispositif optique (31, 32, 33, 34, 35, 36) comporte :
▪ un premier ensemble de dispositifs optiques (31, 32, 33, 34) comportant un premier champ de vue compris entre 30° et 70° verticalement et entre 50 et 100° horizontalement et ;
▪ un second ensemble de dispositifs optiques (35, 36) comportant un second champ de vue compris entre 50° et 100° verticalement et entre 30 et 70° horizontalement.

6. Dispositif robotique (1) selon la revendication 1, **caractérisé en ce que** les dispositifs optiques (31, 32, 33, 34, 35, 36) sont agencés pour couvrir un champ de vue (Fov₁) rapporté au système optique (3') ayant une origine virtuelle déplacée hors du plan comportant les origines des dispositifs optiques.

7. Dispositif robotique (1) selon la revendication 1, **caractérisé en ce que** :
▪ au moins deux dispositifs optiques ({32,33}, {31,34}, {35,36}) sont distancés dans un plan horizontal (x₀, y₀) d'un repère cartésien (R₀) d'une distance comprise dans la gamme [30cm et 100cm] ;
▪ au moins deux dispositifs optiques ({36,34}, {36,33}, {33,34}, {35,31}, {35,32}, {31,32}) sont distancés selon un axe vertical (x₀, y₀) d'un repère cartésien (R₀) d'une distance comprise dans la gamme [30cm et 100cm] ;

8. Dispositif robotique (1) selon la revendication 1, **caractérisé en ce que** le bâti comprend un écran (4) permettant d'afficher une image 3D du corps d'un individu à partir d'au moins un dispositif optique.

9. Dispositif robotique (1) selon la revendication 1, **caractérisé en ce que** le bâti comprend un corps principal (21) et une embase (23), ledit corps principal (21) maintenant le système optique (3') et ladite embase étant pourvue de moyens de déplacement pour rendre le dispositif robotique mobile.

10. Dispositif robotique (1) selon la revendication 1, **caractérisé en ce qu'**il comprend au moins un dispositif électronique pour générer à partir des images acquises par le système d'acquisition (3') un nuage de points représentant une surface tridimensionnelle du corps d'un patient.

11. Dispositif robotique (1) selon la revendication 1, **caractérisé en ce que** le dispositif électronique est configuré pour générer un nuage de point global (ENS_{G}) à partir d'une pluralité de nuage de points (ENS_{G31}, ENS_{G32}, ENS_{G33}, ENS_{G34}, ENS_{G35}, ENS_{G36}) générés à partir de chaque image 3D acquise par chaque dispositif optique (3), ledit nuage de points global comportant une compilation de points de chaque nuage de points.

12. Dispositif robotique (1) selon la revendication 10, **caractérisé en ce que** le dispositif électronique est configuré pour générer, à partir des images acquises par le système d'acquisition (3') des consignes de guidage de la cinématique du bras robotisé (6).

13. Dispositif robotique (1) selon la revendication 10, **caractérisé en ce que** le dispositif électronique est configuré pour générer un premier modèle 3D (MOD_P) d'une surface de tout ou partie d'un corps humain, ledit premier modèle 3D (MOD_P) comportant un maillage (M1, M2), ledit premier modèle 3D étant utilisé pour calculer une première trajectoire (t₁) de guidage à la surface du premier modèle 3D, et pour calculer une première cinématique (c₁) du bras robotisé (6)

14. Dispositif robotique (1) selon la revendication 12 ou 13, **caractérisé en ce que** le dispositif électronique est configuré pour générer en temps réel, à partir des images acquises par le système d'acquisition (3'), un nouveau modèle 3D (MOD_P) d'une surface de tout ou partie d'un corps humain, ledit modèle 3D (MOD_P) comportant un maillage (M1, M2), ledit modèle 3D généré à des instants réguliers étant utilisé pour recalculer en temps réel une nouvelle trajectoire (Nt) de guidage à la surface du modèle 3D, et pour recalculer en temps réel une nouvelle cinématique (Nc) du bras robotisé (6).

15. Dispositif robotique (1) selon la revendication 1, **caractérisé en ce qu'**il est configuré pour mettre en oeuvre une autocalibration des dispositifs optiques du système d'acquisition (3') par rapport à un dispositif optique de référence dudit système d'acquisition (3').

## Patentansprüche

1. Robotische Vorrichtung (1) zur automatischen Führung eines Roboterarms (6), umfassend ein Gestell (2), das einen Roboterarm (6) hält, der gemäß mehrerer Freiheitsachsen angelenkt ist, wobei der Roboterarm (6) eine distale Bedienervorrichtung (8) zur Erzeugung einer Mensch-Maschine-Interaktion aufweist, wobei die robotische Vorrichtung (1) ferner ein Bilderfassungssystem (3') aufweist, das mindestens zwei optische Vorrichtungen (3, 31, 32, 33, 34, 35, 36) aufweist, die fest mit dem Gestell (2) verbunden sind und in mindestens zwei unterschiedlichen Positionen des Gestells (2) eingerichtet sind, wobei jede optische Vorrichtung (3) so konfiguriert ist, dass sie ein 3D-Bild erfasst, wobei das Bilderfassungssystem (3') so konfiguriert ist, dass es eine Vielzahl von 3D-Bilder eines menschlichen Körpers eines Patienten erfasst, die von mindestens den beiden optischen Vorrichtungen (3) kommen, wobei die robotische Vorrichtung (1) eine Recheneinheit zum Generieren in Echtzeit eines menschlichen Körpermodells (MOD_S) des Patienten ausgehend von den erfassten 3D-Bildern und einer Führungsbahn (TRAJ), die auf die Oberfläche des menschlichen Körpermodells (MOD_S) referenziert ist, aufweist, wobei die Bewegungen des Roboterarms (6) auf der Führungsbahn (TRAJ) gesteuert werden.

2. Robotische Vorrichtung (1) nach Anspruch 1, **dadurch gekennzeichnet, dass**:
▪ mindestens eine erste optische Vorrichtung (31, 32, 33, 34) eine erste lineare Anordnung von mindestens zwei Kameras gemäß einer ersten Achse (A₁) umfasst;
▪ mindestens eine zweite optische Vorrichtung (35, 36) eine zweite lineare Anordnung von mindestens zwei Kameras gemäß einer zweiten Achse (A₂) umfasst.

3. Robotische Vorrichtung (1) nach Anspruch 1, **dadurch gekennzeichnet, dass** mindestens eine optische Vorrichtung (3) ein Mittel zur Ausrichtung der Erfassungsachse (N) durch Betätigung einer mechanischen Verbindung umfasst.

4. Robotische Vorrichtung (1) nach Anspruch 1, **dadurch gekennzeichnet, dass** jede optische Vorrichtung (31, 32, 33, 34, 35, 36) umfasst:
▪ mindestens zwei Infrarotkameras; und/oder
▪ mindestens eine Farbkamera; und/oder
▪ mindestens ein Paar Farbkameras, die so konfiguriert sind, dass sie die 3D-Bilder durch Stereoskopie generieren; und/oder
▪ mindestens drei Kameras, die zwei Infrarotkameras und eine Farbkamera umfassen; und/oder
▪ mindestens einen Infrarotprojektor, der ein Muster projiziert, und zwei Infrarotkameras, die das auf die Oberfläche eines menschlichen Körpers projizierte Muster erfassen;
▪ mindestens einen Lichtprojektor, der strukturierte Lichtmuster projiziert, und mindestens eine Kamera, die diese Muster erfasst; und/oder
▪ eine Flugzeitkamera; und/oder
▪ eine Sender-Empfänger-Laservorrichtung.

5. Robotische Vorrichtung (1) nach Anspruch 1, **dadurch gekennzeichnet, dass** jede optische Vorrichtung (31, 32, 33, 34, 35, 36) aufweist:
▪ eine erste Gruppe optischer Vorrichtungen (31, 32, 33, 34) mit einem ersten Sichtfeld zwischen 30° und 70° vertikal und zwischen 50° und 100° horizontal; und
▪ eine zweite Gruppe optischer Vorrichtungen (35, 36) mit einem zweiten Sichtfeld zwischen 50° und 100° vertikal und zwischen 30° und 70° horizontal.

6. Robotische Vorrichtung (1) nach Anspruch 1, **dadurch gekennzeichnet, dass** die optischen Vorrichtungen (31, 32, 33, 34, 35, 36) so angeordnet sind, dass sie ein Sichtfeld (Fov₁) bezogen auf das optische System (3') abdecken, das einen virtuellen Ursprung hat, der außerhalb der Ebene versetzt ist, die die Ursprünge der optischen Vorrichtungen aufweist.

7. Robotische Vorrichtung (1) nach Anspruch 1, **dadurch gekennzeichnet, dass**:
▪ mindestens zwei optische Vorrichtungen ({32,33}, {31,34}, {35,36}) in einer horizontalen Ebene (x₀, y₀) von einem kartesischen Bezugspunkt (R₀) in einem Abstand im Bereich [30 cm und 100 cm] entfernt sind;
▪ mindestens zwei optische Vorrichtungen ({36,34}, {36,33}, {33,34}, {35,31}, {35,32}, {31,32}) gemäß einer vertikalen Achse (x₀, y₀) von einem kartesischen Bezugspunkt (R₀) in einem Abstand im Bereich [30 cm und 100 cm] entfernt sind.

8. Robotische Vorrichtung (1) nach Anspruch 1, **dadurch gekennzeichnet, dass** das Gestell einen Bildschirm (4) umfasst, mit dem ein 3D-Bild des Körpers einer Person ausgehend von mindestens einer optischen Vorrichtung angezeigt werden kann.

9. Robotische Vorrichtung (1) nach Anspruch 1, **dadurch gekennzeichnet, dass** das Gestell einen Hauptkörper (21) und einen Sockel (23) umfasst, wobei der Hauptkörper (21) das optische System (3') hält und der Sockel mit Verschiebemitteln versehen ist, um die robotische Vorrichtung beweglich zu machen.

10. Robotische Vorrichtung (1) nach Anspruch 1, **dadurch gekennzeichnet, dass** sie mindestens eine elektronische Vorrichtung umfasst, um aus den vom Erfassungssystem (3') erfassten Bildern eine Punktwolke zu generieren, die eine dreidimensionale Oberfläche des Körpers eines Patienten darstellt.

11. Robotische Vorrichtung (1) nach Anspruch 1, **dadurch gekennzeichnet, dass** die elektronische Vorrichtung so konfiguriert ist, dass sie eine globale Punktwolke (ENS_{G}) aus einer Vielzahl von Punktwolken (ENS_{G31}, ENS_{G32}, ENS_{G33}, ENS_{G34}, ENS_{G35}, ENS_{G36}) generiert, die aus jedem von jeder optischen Vorrichtung (3) erfassten 3D-Bild generiert werden, wobei die globale Punktwolke eine Punktesammlung jeder Punktwolke aufweist.

12. Robotische Vorrichtung (1) nach Anspruch 10, **dadurch gekennzeichnet, dass** die elektronische Vorrichtung so konfiguriert ist, dass sie aus den vom Erfassungssystem (3') erfassten Bildern Führungsvorgaben für die Kinematik des Roboterarms (6) generiert.

13. Robotische Vorrichtung (1) nach Anspruch 10, **dadurch gekennzeichnet, dass** die elektronische Vorrichtung so konfiguriert ist, dass sie ein erstes 3D-Modell (MOD_P) einer Oberfläche eines ganzen oder eines Teils eines menschlichen Körpers generiert, wobei das erste 3D-Modell (MOD_P) ein Netz (M1, M2) umfasst, wobei das erste 3D-Modell zur Berechnung einer ersten Führungsbahn (t₁) auf der Oberfläche des ersten 3D-Modells und zur Berechnung einer ersten Kinematik (c₁) des Roboterarms (6) verwendet wird.

14. Robotische Vorrichtung (1) nach Anspruch 12 oder 13, **dadurch gekennzeichnet, dass** die elektronische Vorrichtung so konfiguriert ist, dass sie aus den vom Erfassungssystem (3') erfassten Bildern in Echtzeit ein neues 3D-Modell (MOD_P) einer Oberfläche eines ganzen oder eines Teils eines menschlichen Körpers generiert, wobei das 3D-Modell (MOD_P) ein Netz (M1, M2) umfasst, wobei das zu regelmäßigen Zeitpunkten generierte 3D-Modell verwendet wird, um in Echtzeit eine neue Führungsbahn (Nt) auf der Oberfläche des 3D-Modells neu zu berechnen, und um in Echtzeit eine neue Kinematik (Nc) des Roboterarms (6) zu berechnen.

15. Robotische Vorrichtung (1) nach Anspruch 1, **dadurch gekennzeichnet, dass** sie so konfiguriert ist, dass sie eine Selbstkalibrierung der optischen Vorrichtungen des Erfassungssystems (3') in Bezug auf eine optische Referenzvorrichtung des Erfassungssystems (3') durchführt.

## Claims

1. Robot device (1) ensuring the automatic guiding of a robotic arm (6), comprising a frame (2) holding a robotic arm (6) articulated along a plurality of axes of freedom, said robotic arm (6) comprising a distal operator device (8) for generating a human-machine interaction, said robot device (1) further comprising an image acquisition system (3') comprising at least two optical devices (3, 31, 32, 33, 34, 35, 36) integral with said frame (2) and arranged in at least two distinct positions of the frame (2), each optical device (3) being configured to acquire a 3D image, the image acquisition system (3') being configured to acquire a plurality of 3D images of a human body of a patient from at least two optical devices (3), the robot device (1) comprising a calculation unit for generating in real time a human body model (MOD_S) of said patient from said acquired 3D images and a guiding trajectory (TRAJ) referenced to the surface of said human body model (MOD_S), the movements of said robotic arm (6) being enslaved to said guiding trajectory (TRAJ).

2. Robot device (1) according to claim 1, **characterized in that**:
▪ at least one first optical device (31, 32, 33, 34) comprises a first linear arrangement of at least two cameras along a first axis (A₁);
▪ at least one second optical device (35, 36) comprises a second linear arrangement of at least two cameras along a second axis (A₂).

3. Robot device (1) according to claim 1, **characterized in that** at least one optical device (3) comprises a means of orienting the acquisition axis (N) by actuating a mechanical link.

4. Robot device (1) according to claim 1, **characterized in that** each optical device (31, 32, 33, 34, 35, 36) comprises:
▪ at least two infrared cameras and/or;
▪ at least one color camera and/or;
▪ at least one pair of color cameras configured to generate 3D images by stereoscopy and/or;
▪ at least three cameras comprising two infrared cameras and one color camera and/or;
▪ at least one infrared projector projecting a pattern and two infrared cameras capturing said pattern projected onto the surface of a human body.
▪ at least one light projector projecting patterns of structured light and at least one camera capturing said patterns; and/or
▪ a time-of-flight camera and/or;
▪ a laser transceiver device.

5. Robot device (1) according to claim 1, **characterized in that** each optical device (31, 32, 33, 34, 35, 36) comprises:
▪ a first set of optical devices (31, 32, 33, 34) comprising a first field of view between 30° and 70° vertically and between 50 and 100° horizontally; and
▪ a second set of optical devices (35, 36) having a second field of view between 50° and 100° vertically and between 30 and 70° horizontally.

6. Robot device (1) according to claim 1, **characterized in that** the optical devices (31, 32, 33, 34, 35, 36) are arranged to cover a field of view (Fov₁) related to the optical system (3') having a virtual origin displaced out of plane comprising the origins of the optical devices.

7. Robot device (1) according to claim 1, **characterized in that**:
▪ at least two optical devices ({32,33}, {31,34}, {35,36}) are distanced in a horizontal plane (x₀, y₀) of a Cartesian coordinate system (R₀) with a distance comprised in the range [30 cm and 100cm] ;
▪ at least two optical devices ({36,34}, {36,33}, {33,34}, {35,31}, {35,32}, {31,32}) are distanced in a vertical axis (x₀, y₀) from a Cartesian coordinate system (R₀) with a distance between the range [30 cm and 100 cm];

8. Robot device (1) according to claim 1, **characterized in that** the frame comprises a screen (4) for displaying a 3D image of the body of an individual from at least one optical device.

9. Robot device (1) according to claim 1, **characterized in that** the frame comprises a main body (21) and a base (23), said main body (21) holding the optical system (3') and said base being provided with means of displacement to make the robot device movable.

10. Robot device (1) according to claim 1, **characterized in that** it comprises at least one electronic device for generating from the images acquired by the acquisition system (3') a point cloud representing a three-dimensional surface of a patient's body.

11. Robot device (1) according to claim 1, **characterized in that** the electronic device is configured to generate a global point cloud (ENS_{G}) from a plurality of point clouds (ENS_{G31}, ENS_{G32}, ENS_{G33}, ENS_{G34}, ENS_{G35}, ENS_{G36}) generated from each 3D image acquired by each optical device (3), said global point cloud comprising a compilation of points of each point cloud.

12. Robot device (1) according to claim 10, **characterized in that** the electronic device is configured to generate, from the images acquired by the acquisition system (3'), instructions for guiding the kinematics of the robotic arm (6).

13. Robot device (1) according to claim 10, **characterized in that** the electronic device is configured to generate a first 3D model (MOD_P) of a surface of all or part of a human body, said first 3D model (MOD_P) comprising a mesh (M1, M2), said first 3D model being used to calculate a first guiding trajectory (t₁) on the surface of the first 3D model, and to calculate a first kinematic (c₁) of the robotic arm (6)

14. Robot device (1) according to claim 12 or 13, **characterized in that** the electronic device is configured to generate in real time, from the images acquired by the acquisition system (3'), a new 3D model (MOD_P) of a surface of all or part of a human body, said 3D model (MOD_P) comprising a mesh (M1, M2), said regularly generated 3D model being used to recalculate in real time a new guiding trajectory (Nt) on the surface of the 3D model, and to recalculate in real time a new kinematic (Nc) of the robotic arm (6).

15. Robot device (1) according to claim 1, **characterized in that** it is configured to implement self-calibration of the optical devices of the acquisition system (3') with respect to a reference optical device of said acquisition system (3').
